# EUROPEAN PATENT APPLICATION

(11) **EP 2 143 802 A1**
(43) Date of publication of application: **13.01.2010**
(21) Application number: 08738951.6
(22) Date of filing: 26.03.2008
(51) Int. Cl.: C12P 7/06, C12R 1/865

(54) **METHOD FOR CONTINUOUSLY PRODUCING ALCOHOL**

(30) Priority: 30.03.2007 JP 2007095728
(71) Applicant: Mitsui Engineering and Shipbuilding Co, Ltd., Tokyo 104-8439 (JP)
(72) Inventor: TOHYAMA, Masayuki, Ichihara-shi Chiba 290-8601 (JP); KATAGIRI, Manabu, Ichihara-shi Chiba 290-8601 (JP); TAKAOKA, Kazue, Ichihara-shi Chiba 290-8601 (JP)
(74) Representative: Gulde Hengelhaupt Ziebig & Schneider
(86) International application number: PCT/JP2008/055766
(87) International publication number: WO 2008/120644

(57) **Abstract**

[Object]

The invention provides a method for continuous production of an alcohol with cell recycle which exhibits a high ethanol productivity by specifying optimum ranges of recycle ratio and the dilution rate.

[Solution Means]

A method for continuous production of an alcohol including:
placing a yeast having flocculating and settling properties into a fermenter, and feeding an alcohol raw material to the fermenter to conduct alcoholic fermentation; the alcoholic fermentation being conducted while conducting cell recycle by withdrawing the resulting alcohol culture from the fermenter, and recovering yeast cells in the withdrawn culture to return the yeast cells into the fermenter; and creating a forced liquid flow in a lower portion of the fermenter during the alcoholic fermentation, wherein:
an alcohol productivity (g/L·h) is 15 g/Lh or more when the cell recycle is conducted at a cell recycle ratio (r) of 0 < r < 0.4, and a dilution rate D (h⁻¹) of 0.2 ≤ D ≤ 0.3.

## Description

### Technical Field

The present invention relates to a method for continuous production of an alcohol, and more particularly to a method for continuous production of an alcohol with recycle of yeast cells, using a yeast with flocculating and settling properties.

### Background Art

With regard to methods for production of an alcohol, when there is a sufficient amount of an alcohol raw material in producing an alcohol from microorganisms, the amount of cells and the amount of alcohol produced are proportional to a certain extent; therefore, in order to improve the fermentation productivity, it is necessary to maintain a high cell concentration in the fermenter during the operation.

It is preferable that the contents in the fermenter be completely mixed by agitation or the like, because the larger the number of contact times between the alcohol raw material and the yeast, the higher the reaction efficiency becomes. When complete mixing is performed, however, a large amount of yeast is removed together when the alcohol culture is withdrawn from the fermenter, causing the cell concentration in the fermenter to decrease.

If the amount of yeast growth is small relative to the amount of the withdrawn alcohol culture, the amount of the cell outflow cannot be compensated for. Therefore, the amount of the yeast cells present in the fermenter decreases, causing a substantial decrease in the number of contact times between the alcohol raw material and the yeast, resulting in a decrease in the efficiency of the alcohol-producing reaction.

On the other hand, if the contents in the fermenter are not completely agitated, the yeast having flocculating and settling properties will settle and deposit on the bottom of the fermenter. When the settling of the cells proceeds, the settling zone will change to a compaction zone after a lapse of a predetermined time, resulting in the formation of a layer zone (compaction zone) at the bottom. When an alcohol raw material is fed from the bottom of the fermenter, the alcohol raw material passes through the layer zone and come into contact with the yeast. However, the contact takes place in the compacted layer zone where the amount of yeast cells is in excess of the amount of the alcohol raw material; therefore, the efficiency of the reaction between the alcohol raw material and the yeast is extremely low.

Thus, a method is known wherein microorganism cells are recovered from the fermentation broth after the completion of alcoholic fermentation, and the collected cells are recycled.

Patent Literature 1 discloses a method for continuous production of an alcohol with cell recycle. In this method, a fermenter for conducting cell growth and low-concentration alcoholic fermentation and a settling tank for separating cells therefrom, and a fermenter for conducting high-concentration alcoholic fermentation and a settling tank for separating cells therefrom are arranged in series, in such a manner that the cells from each of the settling tanks are returned to each of the two fermenters to give suitable cell concentrations. Continuous production of an alcohol is thus conducted while maintaining and controlling the cell concentrations.

Patent Literature 2 discloses a method for producing an alcohol using a yeast strain *Saccharomyces cerevisiae* AM12 (hereinafter referred to as "strain AM12", as needed), and discloses that AM12 cells possess excellent alcohol productivity and alcohol tolerance, as well as flocculating and settling properties.
Patent Literature 1: Japanese Unexamined Patent Publication No. 6-284891
Patent Literature 2: Japanese Unexamined Patent Publication No. 59-135896

### Disclosure of the Invention

### Problems to be Solved by the Invention

The method of Patent Literature 1, however, requires two fermenters and two settling tanks, resulting in increased facility costs. Further, in the settling tanks, the position for withdrawing the cell culture to be recycled and the position for withdrawing the liquid with a low cell concentration must be controlled according to the circumstance, requiring the trouble of control.

Furthermore, during the cell recycle, even if cells flow out of the fermenter, cells are allowed to settle and separated in order to be returned into the fermenter; therefore, the amount of the cells that flow out of the fermenter is not taken into consideration. If the amount of the cells returned is large, however, the viscosity of the returned cells increases, making the return difficult; thus, it has been found that the amount of the cells that can be recycled is limited.

For this reason, the present inventors conducted continuous production of an alcohol with cell recycle by conducting cell recycle while suppressing the amount of cells that flow out of the fermenter, and consequently obtained a good result.

Accordingly, it is an object of the invention to provide a method for continuous production of an alcohol with cell recycle which exhibits a high ethanol productivity by specifying optimum ranges of the recycle ratio and the dilution rate.

It is another object of the invention to provide a method for continuous production of an alcohol that is capable of preventing the cell outflow to enhance the efficiency of contact between the alcohol raw material and yeast, thereby fully utilizing the alcohol productivity of the yeast having flocculating and settling properties.

Other objects of the invention will become apparent from the following description.

### Means for Solving the Problems

The above-described objects are solved by the inventions set forth below.

The invention as defined in claim 1 is a method for continuous production of an alcohol comprising:
placing a yeast having flocculating and settling properties into a fermenter, and feeding an alcohol raw material to the fermenter to conduct alcoholic fermentation; the alcoholic fermentation being conducted while conducting cell recycle by withdrawing the resulting alcohol culture from the fermenter, and recovering yeast cells in the withdrawn culture to return the yeast cells into the fermenter; and creating a forced liquid flow in a lower portion of the fermenter during the alcoholic fermentation, wherein:
   when the cell recycle is conducted at a cell recycle ratio (r) of 0 < r < 0.4, and a dilution rate D(h⁻¹) of 0.2 ≤ D ≤ 0.3, an alcohol productivity (g/L·h) is 15 g/Lh or more.

The invention as defined in claim 2 is the method for continuous production of the alcohol according to claim 1, wherein the alcoholic fermentation is conducted, with an interface of the yeast with flocculating and settling properties being formed in the fermenter.

The invention as defined in claim 3 is the method for continuous production of the alcohol according to claim 2, wherein the interface can be observed as a boundary of a layer with a high yeast cell concentration and a layer with a low yeast cell concentration that are formed in an alcohol fermentation broth in the fermenter.

The invention as defined in claim 4 is the method for continuous production of the alcohol according to claim 2 or 3, wherein the presence or absence of the interface is visually determined through an observation window formed on a side face of the fermenter.

The invention as defined in claim 5 is the method for continuous production of the alcohol according to any one of claims 2 to 4, wherein the presence or absence of the interface is measured using a fermentation element and a light-receiving element provided above a liquid surface of the fermenter.

The invention as defined in claim 6 is the method for continuous production of the alcohol according to any one of claims 2 to 5, wherein the presence or absence of the interface is detected using a plurality of turbidimeters provided in a height direction downward from the liquid surface of the alcohol fermentation broth in the fermenter.

The invention as defined in claim 7 is the method for continuous production of the alcohol according to any one of claims 1 to 6, wherein the yeast having flocculating and settling properties is *Saccharomyces cerevisiae* strain AM12.

The invention as defined in claim 8 is the method for continuous production of the alcohol according to any one of claims 1 to 7, wherein the average yeast cell concentration in the fermenter is 28 to 178 g (dry weight)/L.

The invention as defined in claim 9 is the method for continuous production of the alcohol according to any one of claims 1 to 8, wherein the forced liquid flow is created in the lower portion of the fermenter principally by means of rotation of an agitator.

### Effects of the Invention

In accordance with the invention, methods for continuous production of an alcohol are provided that are capable of maintaining an alcohol with cell recycle which exhibits a high ethanol productivity by specifying optimum ranges of the recycle ratio and the dilution rate.

Moreover, in accordance with the invention, methods for continuous production of an alcohol are provided that are capable of preventing the cell outflow to enhance the efficiency of contact between the alcohol raw material and the yeast, thereby fully utilizing the alcohol productivity of the yeast having flocculating and settling properties.

### Brief Description of the Drawings

Fig. 1 is a schematic diagram of a fermenter for continuous production of ethanol;
Fig. 2 is a schematic diagram showing the height of a preferable position at which an interface is formed;
Fig. 3 is a schematic diagram showing a position of the interface at which the proportion of the layer (B) with a high yeast cell concentration relative to the volume V of the alcohol fermentation broth is preferable; and
Fig. 4 is a schematic diagram of a fermenter provided with an interface observation window.

### Explanation of Reference Numerals

1: Sugar Solution Feed Tank
   11: Feed Pump
   12: Feed Line
2: Fermenter
   21: Agitator
   22: Solution Withdrawing Pump
   23: Solution Withdrawing Line
   24: Line
3: Settling and Separation Tank
   31: Alcohol Pump
   32: Alcohol Line
   33: Recycle Pump
   34: Recycle Line
   35: Discharge Line
100: Fermenter
   101: Fermenter
   102: Observation Window
   110: Raw Material Inlet
   111: Outlet
   112: Agitator

### Best Mode for Carrying Out the Invention

Embodiments of the present invention will hereinafter be described.

The method for continuous production of an alcohol of the invention is a method for continuous production of an alcohol including placing a yeast having flocculating and settling properties into a fermenter, and feeding an alcohol raw material to the fermenter to conduct alcoholic fermentation; the alcoholic fermentation being conducted while conducting cell recycle by withdrawing the resulting alcohol culture from the fermenter, and recovering yeast cells in the withdrawn culture to return the yeast cells into the fermenter; and creating a forced liquid flow in a lower portion of the fermenter during the alcoholic fermentation.

The purpose of creating a forced liquid flow in the lower portion of a fermenter is to increase the number of contact times between the fermentation raw material and the yeast to increase the efficiency of the fermentation reaction. However, when a forced liquid flow is created in a fermenter by agitation or the like, the cells float upward and may flow out of the fermenter together with the fermentation broth. The yeast with flocculating and settling properties used in the invention inherently exhibits significant flocculating and settling properties, so that the cell concentration near the liquid surface in the fermenter becomes low, which inhibits the cell outflow from the fermenter.

The present inventors used a yeast with flocculating and settling properties in a method for continuous production of an alcohol, and adjusted the forced liquid flow in the lower portion of the fermenter. As a result, the cells floated upward by the liquid flow induced by agitation or the like, while they flocculated and settled due to the flocculating and settling properties of the yeast. Consequently, the boundary between these layers could be observed as an interface.

The term "continuous production of an alcohol" as used herein means that a sugar solution, which is an alcohol raw material, is, for example, continuously fed into a fermenter, while the product is, for example, continuously withdrawn from the fermenter. Therefore, the alcohol raw material may be discontinuously fed as long as the amount of feed per unit time is constant.

The definition of the phrase "with cell recycle" as used herein is that, during the alcoholic fermentation, the culture containing ethanol and yeast that has been withdrawn from the fermenter is allowed to stand, allowing the separation of yeast cells by sedimentation, and a portion of the separated cells are returned into the fermenter, regardless of whether the cells are re-cultured or not.

In the invention, when the cell recycle is conducted at a cell recycle ratio (r) of 0 <r < 0.4, and at a dilution rate D (h⁻¹) of 0.2 ≤ D ≤ 0.3, a high ethanol productivity can be maintained such that the alcohol productivity (g/L·h) is 15 g/Lh or more. That is, the present inventors found the range of recycle ratio and the range of dilution rate in which the yeast used in the invention exhibits a high ethanol productivity.

The recycle ratio (r) is represented by the ratio (by weight or volume) of the amount of cells to be recycled relative to the total amount of the recovered cells, and the dilution rate D is a result of dividing the feed rate of the saccharified solution by the culture volume. The ethanol productivity is determined as the product of the ethanol concentration in the fermenter and the dilution rate D.

The inventors found the following: When a cell interface is formed in the fermenter during the operation under the above-described conditions, the region below the interface having a high cell concentration is different from the compaction zone formed by flocculation and settling of the cells; the cell concentration below the interface and that above the interface are extremely different. With this interface being formed, the fermentation reaction of the alcohol raw material is promoted rather than inhibited. The inventors also found that when such an interface is formed under agitation, the cell outflow can be prevented by withdrawing the fermentation broth present above the interface to enhance the efficiency of contact between the alcohol raw materials and yeast, thereby fully utilizing the alcohol productivity of the AM12 cells.

In the continuous production of an alcohol of the invention, a small amount of cells may possibly flow out of the fermenter although the outflow of the cells from the fermenter is inhibited. However, any decrease in the cell concentration in the fermenter due to the cell outflow is prevented. In the invention, the amount of cell growth in the fermenter is larger than the amount of cell outflow, because a suitable balance of concentrations is maintained vertically in the fermenter; thus, the invention does not basically intend to constantly add the cells into the fermenter. However, the effects of the invention are by no means impaired by adding the cells as an inoculum, or by separating the small amount of cells that have flown out of the fermenter, and returning the cells into the fermenter.

The alcohol raw material used in the invention is preferably a raw material derived from grains. Preferable examples include those from rice, wheat, and potatos, because of their high sugar concentrations. In view of efficient saccharification reaction of the grain raw material, the sugar concentration is preferably 100 to 300 g/L, and more preferably 150 to 200 g/L, when calculated as glucose.

In view of an optimum fermentation temperature of the yeast, the aerobic fermentation temperature is preferably 40°C or less, and more preferably 30 to 35°C. The temperature may be controlled by employing a control means such as a heater or a cooler, but the means for controlling the temperature is not limited thereto.

In the invention, DO (Dissolved Oxygen Concentration) in the fermenter is preferably 0 to 3 ppm, and more preferably 0 to 0.1 ppm. Depending on the growth condition of the cells, DO may be zero. DO can be adjusted by increasing or decreasing the amount of air fed and the rotation speed of the impeller in the fermenter. For example, a method of interlocking a DO meter with a means for controlling the amount of air fed can be employed.

In view of an optimum pH of the yeast, the pH is preferably 3 to 7, and more preferably 3.5 to 5.0. The pH can be adjusted by employing a pH control system that adjusts the pH by continuously feeding a pH adjuster based on the data measured using a pH meter.

The yeast with flocculating and settling properties may be any yeast having settling properties such that an interface can be formed even during agitation. Yeast strain *Saccharomyces cerevisiae* AM12 or yeast strain *Saccharomyces cerevisiae* TJ1 is preferably used.

Both strains AM12 and TJ1 have been deposited with National Institute of Bioscience and Human Technology, Microorganism Research Institute (currently Advanced Industrial Science and Technology, International Patent Organism Dipositary (IPOD)) under the accession numbers 6749 and 6747, respectively, and are available therefrom.

Embodiments of the invention are hereinafter described with reference to the drawings.

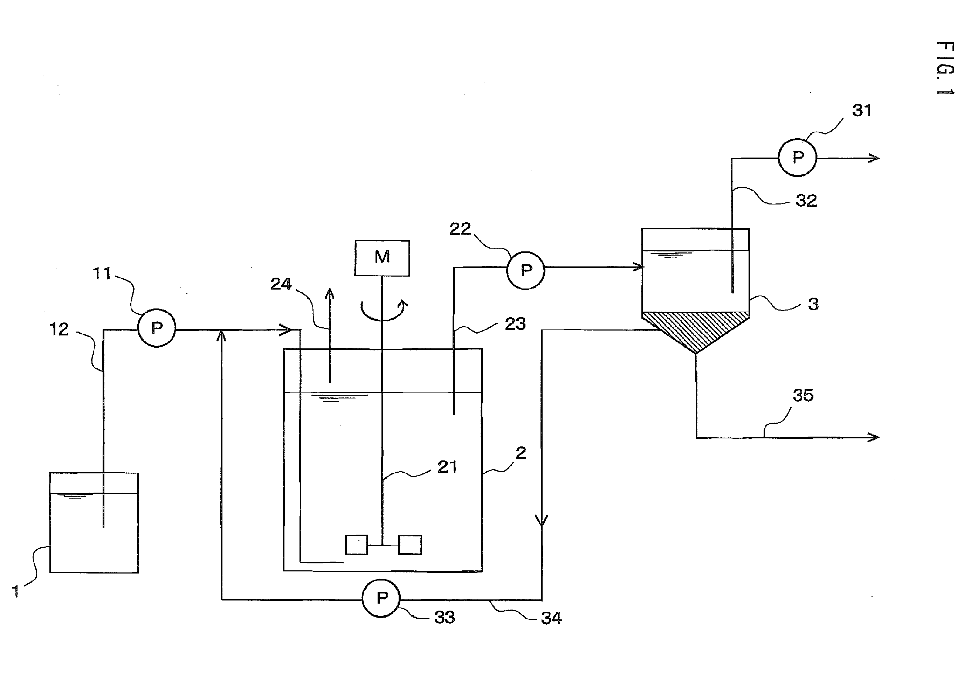

Referring to Fig. 1, reference numeral 1 denotes a sugar solution feed tank, reference numeral 2 denotes a fermenter, and reference numeral 3 denotes a settling and separation tank, which is an apparatus for separating cells.

The raw material is fed from the sugar solution feed tank 1 to the fermenter 2 via a feed pump 11 and a feed line 12. In addition to the sugar solution as a raw material, a nutrient solution necessary for cell growth including peptone, a solution containing a small amount of a metal salt, and the like can be added via the feed line 12.

The fermenter 2 incorporates therein an agitator 21 for sufficiently mixing the cells and the raw material fed. The agitator may be any that can sufficiently mix the contents of the fermenter. An agitator with an impeller such as a propeller is preferably used. The number of revolutions of the agitator is adjusted to be optimum for the yeast. Although not shown in the figure, the fermenter preferably has a system for maintaining the pH, temperature, and dissolved oxygen concentration; such a system is not particularly limited.

In the fermenter 2, alcoholic fermentation is conducted by the yeast, and the raw material sugar solution is converted to a liquid containing an alcohol and yeast cells, which is then delivered to the settling and separation tank 3 via a solution withdrawing pump 22 and a solution withdrawing line 23.

Gas produced in alcoholic fermentation is discharged via a line 24.

The settling and separation tank 3 allows the liquid to stand, allowing settling and separation of the cells. The settled flocculating yeast is separated from the alcohol. The alcohol is delivered to the subsequent purification process via an alcohol pump 31 and an alcohol line 32.

A portion of the settled and concentrated cells are withdrawn, and a predetermined amount of the cells that is determined from the recycle ratio are returned into the fermenter 1 from the feed line 12, together with the raw material, via a recycle line 34 that is connected to a recycle pump 33 and a feed line 12.

Excess sediment is discharged via a discharge line 35.

In the invention, the yeast with flocculating and settling properties is used, thereby enabling continuous production of ethanol that is efficient and exhibits a high alcohol productivity.

In the invention, the recycle ratio (r) at which cells are returned is preferably 0 < r < 0.4, more preferably 0 < r < 0.3, and still more preferably 0 < r < 0.22.

A recycle ratio of 0 is not effective in the invention because cell recycle cannot be conducted. A recycle ratio exceeding 0.4 will make the cell concentration in the fermenter excessively high, causing the ethanol fermentation ability of the yeast cells returned into the fermenter to decrease, or causing cell death, and also causing the viscosity of the returned solution to increase, making the return difficult.

The dilution rate D (h⁻¹) is preferably 0.2 ≤ D ≤ 0.3, and more preferably 0.2 ≤ D ≤ 0.25.

A dilution rate D of less than 0.2 will result in a small effect of the continuous alcoholic fermentation with cell recycle. A dilution rate D exceeding 0.3 will result in excess sugar solution that is fed to the yeast, so that the fermentation broth that is withdrawn from the solution withdrawing line 23 will contain residual sugar that has not been consumed (residual sugar). The residual sugar is an unreacted raw material, and thus becomes a cause of lowering the ethanol productivity (fermentation yield).

During the operation under these conditions, a sufficient cell concentration is maintained in the fermenter, leading to production of high-concentration ethanol.

The ethanol productivity that is determined as the product of the ethanol concentration in the fermenter and the dilution rate D is at least 15 g/L.h or more, and generally 20 g/L.h.

Next, a preferred method of attaining the above-described ethanol productivity is described.

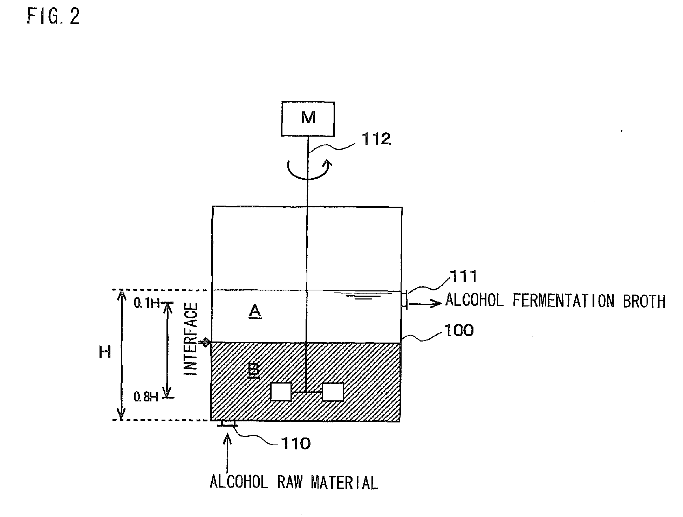

Fig. 2 shows an example of producing an alcohol with an interface being formed in the fermenter. In Fig. 2, reference numeral 100 denotes a fermenter, and an alcohol raw material and the cells to be returned are fed via a raw material inlet 110 at the bottom of the fermenter 100. A yeast with flocculating and settling properties has been placed in the fermenter 100.

When the alcohol raw material is, for example, continuously fed into the fermenter 100 via the raw material inlet 110, alcoholic fermentation is conducted by the yeast to produce an alcohol fermentation broth. The alcohol fermentation broth is withdrawn via an outlet 111. The location of the raw material inlet 110 is shown only by way of example, and the alcohol raw material may be fed by any method as long as it is fed via the bottom of the fermenter. Similarly, the alcohol fermentation broth can be withdrawn by any method as long as it is withdrawn via an upper portion of the fermenter.

An important feature of the invention is that alcoholic fermentation is conducted, with an interface being formed in the alcohol fermentation broth in the fermenter 100, even when a liquid flow is being induced by agitation or the like.

The term "interface" means a linear boundary of the surface that is observed in the alcohol fermentation broth, and formed at the boundary between two layers that differ in turbidity (transparency) or hue. The linear boundary is observed when the fermenter is viewed horizontally, and may not be necessarily straight.

These layers are discernible as two layers that differ in turbidity (transparency) or hue, because they have dramatically different yeast cell concentrations in the alcohol fermentation broth.

For example, above the interface, the turbidity is low, and the color of the alcohol fermentation broth itself is observed, while below the interface is cloudy and has a high turbidity because a large amount of yeast cells are present. Thus, the difference between the layers A and B is clear, allowing the presence or absence of the interface to be visually observed.

The layer (A) above the interface is a layer with an extremely low concentration of yeast cells, and the layer (B) below the interface is a layer with a high concentration of yeast cells.

Cell concentrations can be quantitatively expressed as follows. The average yeast cell concentration in the alcohol fermentation broth in the fermenter, which is a cell concentration measured when the alcohol fermentation broth is in the form of a complete mixture, and is equal to the result of dividing the total amount of yeast cells (dry weight: g) in the fermenter by the amount of the alcohol fermentation broth (L) in the fermenter, is preferably 28 to 178 (dry weight)/L, more preferably 50 to 120 g (dry weight)/L, and still more preferably 70 to 100 g (dry weight)/L. Comparatively, the layer (A) with a low yeast cell concentration above the interface has a yeast cell concentration of preferably less than 17 g (dry weight)/L, more preferably less than 14 g (dry weight)/L, and still more preferably less than 10 g (dry weight)/L; and the layer (B) with a high yeast cell concentration below the interface has a yeast cell concentration of preferably 35 to 180 g (dry weight)/L, more preferably 55 to 150 g (dry weight)/L, and still more preferably 75 to 120 g(dry weight)/L.

It is important that the interface be formed below the outlet 111 for withdrawing the alcohol fermentation broth. This allows the layer with a low yeast cell concentration of the alcohol fermentation broth to be withdrawn from the fermenter, thereby inhibiting the outflow of yeast cells.

Due to their different yeast cell concentrations, the layer (B) with a high yeast cell concentration naturally has an alcohol productivity higher than that of the layer (A) with a low yeast cell concentration. Therefore, the higher the proportion of the layer (B) with a high yeast cell concentration that is present below the interface is, the higher the productivity becomes.

Specifically, the interface is formed in a region with a depth of 0.1 to 0.8 H (H: liquid surface height), preferably 0.2 to 0.6 H, and more preferably 0.2 to 0.5 H, downward from the liquid surface of the alcohol fermentation broth in the fermenter. Alternatively, the layer (B) with a high yeast cell concentration accounts for 20 to 90%, preferably 40 to 80%, and still more preferably 50 to 80%, of the volume V of the alcohol fermentation broth, and is preferably formed at the bottom side (in the lower portion) of the fermenter.

Fig. 2 shows a preferable position at which the interface is formed in the height direction.

In a fermenter having a constant cross-sectional area, such as a cylindrical fermenter, the position at which the interface is observed is defined within a region with a depth of 0.1 to 0.8 H (H: liquid surface height), preferably 0.2 to 0.6 H, and more preferably 0.2 to 0.5 H, downward from the liquid surface of the alcohol fermentation broth in the fermenter.

When the interface is formed in a region with a depth of 0.1 to 0.8 H, preferably 0.2 to 0.6 H, and more preferably 0.2 to 0.5 H, there is a good balance between agitation and settling, so that a high cell concentration in the fermenter 100 is maintained, and the efficiency of contact between the yeast cells and the alcohol raw material is also high, resulting in a high ethanol productivity.

When the position of the interface is above 0.1 H, the alcohol fermentation broth is nearly in the form of a complete mixture. This is undesirable because the cells easily flow out of the outlet 111 to cause the amount of the cells in the fermenter to decrease, which makes the alcohol productivity lower.

When the position of the interface is below 0.8 H, the cells in the fermenter begin to deposit on the bottom of the fermenter, forming a settling zone or a compaction zone. In the settling zone or compaction zone, yeast is present in an amount larger than the alcohol raw material, even though the alcohol raw material is being fed via the bottom of the fermenter. This is undesirable because the efficiency of the reaction between the alcohol raw material and the yeast is extremely low, which also makes the alcohol productivity lower.

The interface cannot be observed if, for example, the alcohol fermentation broth is completely mixed, or the amount of the cells in the fermenter is too small to discern a difference in concentration. Both of these cases are undesirable in carrying out the methods for the production of an alcohol of the invention.

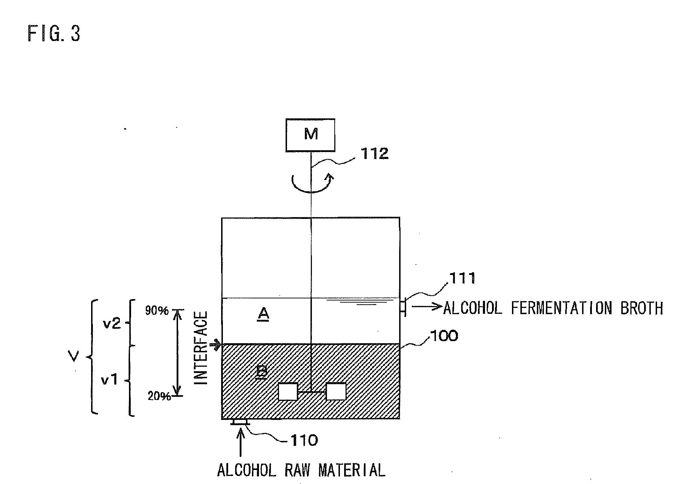

Fig. 3 is a schematic diagram showing a position of the interface at which the proportion of the layer (B) with a high yeast cell concentration relative to the volume V of the alcohol fermentation broth is preferable. Since the interface is formed at the boundary between the layer (A) with a low yeast cell concentration and the layer (B) with a high yeast cell concentration, the position of the interface can be defined by setting the proportion of the layer (B) with a high yeast cell concentration formed at the bottom side (in the lower portion) of the fermenter to 20 to 90%, preferably 40 to 80%, and more preferably 50 to 80%, relative to the volume V of the alcohol fermentation broth. Assuming that the volume of the layer (B) with a high yeast cell concentration is v1, and the volume of the layer (A) with a low yeast cell concentration is v2, V = v1 + v2.

When the proportion of the layer with a high yeast cell concentration is set in terms of volume, the method of the invention is also applicable to cases where the fermenter has a shape with a variable cross-sectional area, or where the position of the interface is difficult to define in terms of height.

In the invention, when the volume v1 of the layer (B) with a high yeast cell concentration accounts for more than 90% of the volume V of the alcohol fermentation broth, the alcohol fermentation broth is nearly in the form of a complete mixture. This is undesirable because the cells easily flow out of the outlet 111 to cause the amount of the cells in the fermenter to decrease, which makes the alcohol productivity lower.

Conversely, when the volume v1 of the layer (B) with a high yeast cell concentration is lower than 20% of the volume v of the alcohol fermentation broth, the cells in the fermenter begin to deposit on the bottom of the fermenter, forming a settling zone or a compaction zone. In the settling zone or compaction zone, yeast is present in an amount larger than the alcohol raw material, even though the alcohol raw material is being fed via the bottom of the fermenter. This is undesirable because the efficiency of the reaction between the alcohol raw material and the yeast is extremely low, which also makes the alcohol productivity lower.

In the invention, a means for creating a forced liquid flow in the lower portion of the fermenter is provided, in addition to the liquid flow created by feeding the alcohol fermentation broth. The forced liquid flow is preferably created principally by means of rotation of an agitator. Specifically, the forced liquid flow may be created by using an agitator alone, or by using an agitator and aeration in combination, but the use of an agitator alone is preferred in view of the convenience of control.

In the examples of Figs. 2 and 3, an agitator 112 with an impeller is shown as the means for agitation.

The position of the interface can be maintained by suitably varying the operating conditions for continuous fermentation, such as the intensity of agitation, so that the interface can be formed in the above-described preferable region.

The interface rises as the force of the liquid flow to cause the cells to flow upward increases; conversely, the interface begins to settle and fall down as the force of the liquid flow to cause the cells to flow upward decreases.

For example, the interface rises when the rotation of the agitator is increased.

The interface also rises when the amount of the alcohol raw material or the amount of the air fed is increased to cause an upward flow.

When the alcohol fermentation broth is in the form of a complete mixture, the rotation of the agitator may be ceased, or the rotation may be reduced to allow the formation of an interface.

The presence or absence of the interface can be visually observed because of the difference in hue or transparency between the layer (A) with a low yeast cell concentration and the layer (B) with a high yeast cell concentration.

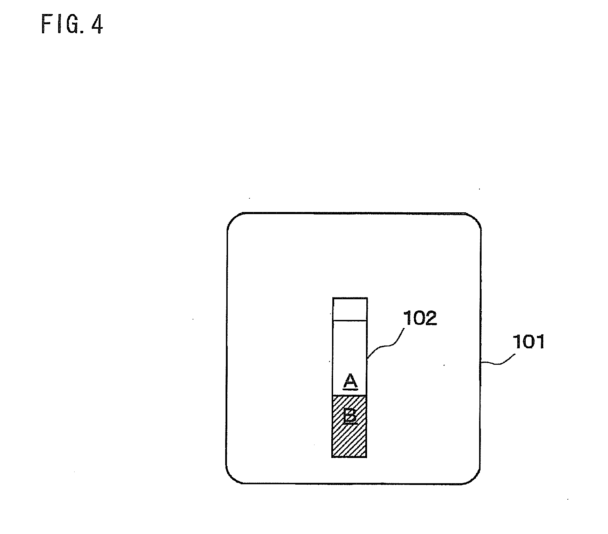

In the case of a fermenter that is not transparent, such as a stainless steel fermenter, the fermenter 101 may be provided with an observation window 102, as shown in Fig. 4, which allows one to abserve the presence or absence of the interface.

Alternatively, the presence or absence of the interface can be confirmed by measuring the cell concentration using two or more turbidimeters provided vertically at different positions downward from the liquid surface of the alcohol fermentation broth in the fermenter.

Still alternatively, the presence or absence of the interface can be measured by using a light-emitting element and a light-receiving element provided above the liquid surface in the fermenter. In this way, the light from the light-emitting element is reflected at the interface, and the reflected light is received by the light-receiving element, to determine the change in the amount of light.

### EXAMPLE

Examples of the present invention are described below; however, the invention is not limited by these Examples.

### Example 1

### <Preparation of Yeast Inoculum Culture>

10 ml of YPD medium (1% yeast extract, 2% peptone, 5% glucose, pH 5.0) was placed in a 200 ml flask and sterilized; the flask was subsequently inoculated with each yeast colony stored using a platinum loop, and incubated for 20 hours under aerobic conditions at 30°C and 200 rpm, to restore the yeast (AM12 cells) to the culturable state.

A 250 ml flask containing 100 ml of sterile YPD medium was inoculated with 10 ml of the restored yeast culture. The yeast was incubated for 20 hours under aerobic conditions at 30°C and 200 rpm, to further grow the yeast inoculum in the logarithmic growth phase.

### <Continuous Fermentation with Jar fermenter>

110 ml of the above-described yeast inoculum culture was inoculated into a jar fermenter containing 900 ml of sterile CSL medium for start-up (1% corn steep liquor (CSL), 0.1% KH₂PO₄, 0.05% MgSO₄·7H₂O, 0.01% CaCl₂·H₂O, 10% glucose, pH 5.0).

The yeast culture was inoculated using a feed pump to prevent exposure to the outside air.

The operating conditions of the jar fermenter were as follows.

pH: 5.0 (adjusted with 2N NaOH and HC1)
Aeration: 0.5 to 1.5 vvm (aeration was provided by passing air from a compressor via a sterile filter)
Agitation Speed: 50 to 150 rpm
Temperature: 30°C

During the operation, the culture was sampled from the fermenter after 24 and 48 hours from the start of the fermentation, and the glucose concentration was measured to confirm that the fermentation broth had a sugar concentration of 5 g/L.

Sterile CSL medium for continuous production (1% CSL, 0.1% KH₂PO₄, 0.05% MgSO₄·7H₂O, 0.01% CaCl₂·H₂O, 20% glucose, pH 5.0) was continuously fed into the jar fermenter, while the culture (alcohol fermentation broth) was withdrawn at the same rate as the medium fed, with the rotation of an agitator being adjusted so that an interface is formed. Continuous fermentation was thus started (the start of continuous fermentation). The withdrawn culture was stored in 3 L flocculation and settling tank, and the portion of the culture that exceeded 3 L was withdrawn from the liquid surface.

When analysis of the withdrawn portion of the culture showed that the concentrations of the yeast cells, glucose, and ethanol in the fermenter had reached steady-state values, the yeast cells that settled to the lower portion of the settling tank were returned according to a predetermined recycle ratio; cell recycle was thus started (the start of cell recycle). The yeast growth, the glucose consumption, and the amount of ethanol produced were analyzed according to the following methods.

The yeast growth was measured based on turbidity (absorbance: OD_{600 nm}) to determine the concentration using a conversion factor to the cell concentration (0.37 x OD_{600 nm}).
The glucose consumption was measured using a glucose kit (Glucose Test C Wako; Wako Pure Chemical Ind. Ltd.).
Change over time in ethanol production was measured by analyzing the ethanol concentration in the culture (Shimadzu GC-2014, detector FID).

Under these conditions, the operation was conducted at a cell recycle ratio of 0.1, and a dilution rate of 0.22; as a result, the ethanol productivity was 22.1 g/L·h.

### Example 2

The fermenter was controlled as in Example 1, and the operation was conducted at a cell recycle ratio of 0.3; as a result, the ethanol productivity was 21.9 g/L·h.

### Comparative Example 1

The fermenter was controlled as in Example 1, and the operation was conducted at a cell recycle ratio of 0.5; as a result, despite an average yeast cell concentration of 200.9 g/L in the fermenter, the ethanol productivity was 22.0 g/L.h. Although the cell concentration was more than double that of Example 1 or 2, the ethanol productivity was not substantially different from that of Example 1.

### Comparative Example 2

The fermenter was controlled as in Example 1, and the operation was conducted at a cell recycle ratio of 0.1 and a dilution rate of 0.1; as a result, the ethanol productivity was 10.2 g/L·h.

### Comparative Example 3

The fermenter was controlled as in Example 1, and the operation was conducted at a cell recycle ratio of 0.1 and a dilution rate of 0.4; as a result, the ethanol productivity was 12.1 g/L·h.

### Comparative Example 4

The fermenter was controlled as in Example 1, and the operation was conducted at a cell recycle ratio of 0.1 and a dilution rate of 0.6; as a result, the ethanol productivity was 8.0 g/L·h.

### Example 3

Using strain TJ1 instead of strain AM12, the operation was conducted under the same conditions as Example 1; as a result, the ethanol productivity was 17.6 g/L.h.

### Example 4

The operation was conducted under the same conditions as Example 3, except that the recycle ratio was 0.3; as a result, the ethanol productivity was 16.7 g/L-h.

### Comparative Example 5

The fermenter was controlled as in Example 1, and the operation was conducted at a cell recycle ratio of 0.1 and a dilution rate of 0.1; as a result, the ethanol productivity was 7.48 g/L·h.

### Reference Example 1

Continuous operation was conducted under the same conditions as Example 1, using a yeast that does not exhibit flocculating and settling properties (*Saccharomyces cerevisiae* strain (JCM7255)) instead of the strain AM12; as a result, the ethanol productivity was 10.1 g/L·h.

The above experimental results are shown in Table 1.

**[Table 1]**

| | Operating Conditions | | Experimental Values | | | |
|---|---|---|---|---|---|---|
| | Recycle Ratio | Dilution Rate | Average Cell Concentration (g/L) | Amount of Residual Sugar (g/L) | Average Ethanol Concentration (g/L) | Ethanol Productivity (g/L·hr) |
| Ex. 1 | 0.1 | 0.22 | 76.5 | 0 | 100.45 | 22.1 |
| Ex. 2 | 0.3 | 0.22 | 93.0 | 0 | 99.6 | 21.9 |
| Ex. 3 | 0.1 | 0.22 | 73.0 | 0.2 | 79.3 | 17.6 |
| Ex. 4 | 0.3 | 0.22 | 89.6 | 0 | 76.1 | 16.7 |
| Comp. Ex. 1 | 0.5 | 0.22 | 200.9 | 0 | 100.0 | 22.0 |
| Comp. Ex. 2 | 0.1 | 0.1 | 36.2 | 0 | 102.0 | 10.2 |
| Comp. Ex. 3 | 0.1 | 0.4 | 38.9 | 100.6 | 30.3 | 12.1 |
| Comp. Ex. 4 | 0.1 | 0.6 | 20.7 | 152.9 | 13.3 | 8.0 |
| Comp. Ex. 5 | 0.1 | 0.1 | 36.1 | 0 | 74.8 | 7.48 |
| Ref. Ex. 1 | 0.1 | 0.22 | 30.1 | 97.3 | 45.9 | 10.1 |

## Claims

1. A method for continuous production of an alcohol comprising:
placing a yeast having flocculating and settling properties into a fermenter, and feeding an alcohol raw material to the fermenter to conduct alcoholic fermentation; the alcoholic fermentation being conducted while conducting cell recycle by withdrawing the resulting alcohol culture from the fermenter, and recovering yeast cells in the withdrawn culture to return the yeast cells into the fermenter; and creating a forced liquid flow in a lower portion of the fermenter during the alcoholic fermentation, wherein:
when the cell recycle is conducted at a cell recycle ratio (r) of 0 < r < 0.4, and a dilution rate D(h⁻¹) of 0.2 ≤ D ≤ 0.3, an alcohol productivity (g/L.h) is 15 g/Lh or more.

2. The method for continuous production of the alcohol according to claim 1, wherein the alcoholic fermentation is conducted, with an interface of the yeast with flocculating and settling properties being formed in the fermenter.

3. The method for continuous production of the alcohol according to claim 2, wherein the interface can be observed as a boundary of a layer with a high yeast cell concentration and a layer with a low yeast cell concentration that are formed in an alcohol fermentation broth in the fermenter.

4. The method for continuous production of the alcohol according to claim 2 or 3, wherein the presence or absence of the interface is visually determined through an observation window formed on a side face of the fermenter.

5. The method for continuous production of the alcohol according to any one of claims 2 to 4, wherein the presence or absence of the interface is measured using a fermentation element and a light-receiving element provided above a liquid surface of the fermenter.

6. The method for continuous production of the alcohol according to any one of claims 2 to 5, wherein the presence or absence of the interface is detected using a plurality of turbidimeters provided in a height direction downward from the liquid surface of the alcohol fermentation broth in the fermenter.

7. The method for continuous production of the alcohol according to any one of claims 1 to 6, wherein the yeast having flocculating and settling properties is *Saccharomyces cerevisiae* strain AM12.

8. The method for continuous production of the alcohol according to any one of claims 1 to 7, wherein the average yeast cell concentration in the fermenter is 28 to 178 g (dry weight)/L.

9. The method for continuous production of the alcohol according to any one of claims 1 to 8, wherein the forced liquid flow is created in the lower portion of the fermenter principally by means of rotation of an agitator.

## Amended claims

### Amended claims under Art. 19.1 PCT

1. (Amended) A method for continuous production of an alcohol comprising:
placing a yeast having flocculating and settling properties, which is *Saccharomyces cerevisiae* strain AM12, into a fermenter, and feeding an alcohol raw material to the fermenter to conduct alcoholic fermentation; the alcoholic fermentation being conducted while conducting cell recycle by withdrawing the resulting alcohol culture from the fermenter, and recovering yeast cells in the withdrawn culture to return the yeast cells into the fermenter; and creating a forced liquid flow in a lower portion of the fermenter during the alcoholic fermentation, wherein:
an alcohol productivity (g/L·h) is 20 g/L·h or more when the cell recycle is conducted at a cell recycle ratio (r) of 0 < r < 0.4, and a dilution rate D (h⁻¹) of 0.2 ≤ D ≤ 0.4.

2. The method for continuous production of the alcohol according to claim 1, wherein the alcoholic fermentation is conducted, with an interface of the yeast with flocculating and settling properties being formed in the fermenter.

3. The method for continuous production of the alcohol according to claim 2, wherein the interface can be observed as a boundary of a layer with a high yeast cell concentration and a layer with a low yeast cell concentration that are formed in an alcohol fermentation broth in the fermenter.

4. The method for continuous production of the alcohol according to claim 2 or 3, wherein the presence or absence of the interface is visually determined through an observation window formed on a side face of the fermenter.

5. (Amended)
The method for continuous production of the alcohol according to any one of claims 2 to 4, wherein the presence or absence of the interface is measured using a light-emitting element and a light-receiving element provided above a liquid surface of the fermenter.

6. The method for continuous production of the alcohol according to any one of claims 2 to 5, wherein the presence or absence of the interface is detected using a plurality of turbidimeters provided in a height direction downward from the liquid surface of the alcohol fermentation broth in the fermenter.

7. (Cancelled)

8. The method for continuous production of the alcohol according to any one of claims 1 to 7, wherein the average yeast cell concentration in the fermenter is 28 to 178 g (dry weight)/L.

9. The method for continuous production of the alcohol according to any one of claims 1 to 8, wherein the forced liquid flow is created in the lower portion of the fermenter principally by means of rotation of an agitator.
